(19) **Europäisches Patentamt**
**European Patent Office**
**Office européen des brevets**

(11) **EP 4 343 771 A1**

(12) **EUROPEAN PATENT APPLICATION**

(43) Date of publication:
**27.03.2024 Bulletin 2024/13**

(21) Application number: **22197848.9**

(22) Date of filing: **26.09.2022**

(51) International Patent Classification (IPC):
***G16B 40/30*** *(2019.01)*

(52) Cooperative Patent Classification (CPC):
**G16B 40/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Mohammed Bin Rashid University of Medicine and Health Sciences Dubai (AE)**

(72) Inventor: **Uddin, Mohammed Dubai (AE)**

(74) Representative: **FRKelly Waterways House Grand Canal Quay Dublin D02 PD39 (IE)**

(54) **A METHOD AND SYSTEM FOR DETECTING CELL TYPES AND THEIR ASSOCIATED MARKERS**

(57) The invention sets out a method of detecting cell types and their associated markers. The invention also sets out a system for detecting cell types and their associated markers.

EP 4 343 771 A1

## Description

### Field of the Invention

This invention relates to method and system for detecting cell types and their associated markers.

### Background to the Invention

In recent years, single cell sequencing has considerably advanced our understanding of biological systems. The revolution occurring in high-throughput technologies has provided us with deeper insights of transcriptomes at a single-cell resolution, enabling a better understanding of cellular heterogeneity. For instance, single-cell RNA sequencing (scRNA-seq) can identify complex and rare cell populations, visualize trajectories of developing distinct cell lineages, and even reveal the gene regulatory network. However, known approaches are applied in low dimensional space which will incur some amount of data loss when compressed into specific number of dimensions Single cell OMICs identified numerous disease associated cell types the last few years. With the advent of high-throughput omics, researchers are presented with the challenges of processing and analysing multidimensional data sets acquired from DNA, RNA, or peptide sequencing and their profiling technologies. It includes information about nucleotide/protein sequences and their expression, interactions, and other related phenotypes.

Most recent prior art approaches involve dimensionality reduction techniques that cause a loss of information regarding variance exists within single cell OMICs data. Generally, when scOMICs data is used to detect complex and rare cell population, cell lineage and gene markers, low dimension data is employed.

In contrast, preferred embodiments of the invention disclosed herein used high dimension data without applying any data reduction method to overcome the problems related to the loss of information.

In the current huge data era there are many challenges when analysing the high dimensional data obtained. Indeed, the problems amplify exponentially when the number of variables (i.e., number of transcripts, level of expression) outnumbers the sample size (i.e., cells, tissues). This phenomenon is often referred to as the 'curse of dimensionality'.

In high dimensional data, there is an increased sparsity of 'zero values' observed which is challenging to overcome and handle. For example, scRNA-seq measurements may contain cells lacking a certain molecular marker or specific mapped reads making it more difficult to acquire a representative data about the population. Also, high correlation may be observed between two or several predictor variables leading to a perfect multicollinearity. Other drawbacks of dimensionality occurring are model overfitting, estimation instability, local convergence and huge estimation errors which negatively compromise the reproducibility of results. These challenges among others called the necessity for advancing computational methods to handle the scale and complexity of high dimensional datasets.

The application of computational methods on voluminous and heterogeneous data requires compression and normalization before being processed. It is known to use statistical methods to reduce the data accurately and efficiently through feature selection or dimensionality reduction. Feature selection offers the selection of the most relevant variables from the original data while dimensionality reduction is done by projecting a lower-dimensional component (k) in a new subspace as a representative of the original high dimensional data (p). Several dimensionality reduction methods were introduced such as principle component analysis (PCA), linear discriminant analysis (LDA), generalized discriminant analysis (GDA) etc., to solve troubles concerning high dimensional data, each suitable for data mining procedures and various types of high dimensional data.

The widely used linear transformation approach to reduce dimensionality in multi-featured data is PCA that aims to present data in a new coordinate system where the limited variables of the dataset can be expressed without a significant error. It transforms data into principal components by establishing a linear correlation among the original features of the data set. However, in an attempt at data compression and standardization, the PCA method results in loss of information, low readability, and interpretability of the original features. Moreover, one of the disadvantages of PCA is the use of normalized data instead of the raw or scaled data, otherwise, the algorithm will fail to find optimal principal components and will be biased towards the components with high variance. Also, the outliers in the data must be removed before processing. The assumption of orthogonality is another limitation to the PCA application. Since there is an orthogonal design among the principal components, the technique fails to decompose the data with non-orthogonal axes. In practice, most studies consider the high variance PCA and ignores most of the low variance PCA that leads to bias towards high value signals (i.e., high expressed feature cluster) and may not capture low signals from the data (i.e., small rare low expressed cell clusters in scRNA-seq or other OMICs data).

Another problem of using PCA is that the resultant cell clusters are not biologically interpretable i.e. they are not be interpretable in light of molecular biology. The PCA derived approach only clusters cells because they have similar variance, but does not specify why this variance exists in the data utilizing molecular biology knowledge. Scientists apply numerous downstream analysis to identify the cause of cell clusters and attempt to establish correlations between cell clusters and biology.

Since applying PCA for dimensionality reduction prior to clustering reduces the cluster, it is typical in prior art methods to avoid dimension reduction methods to prevent the consequences and complexity of missing information from the obtained data and avoid substantial bias in the result interpretations.

It would be desirable to mitigate at least some of the problems outlined above.

## Summary of the Invention

The invention sets out a method of detecting cell types and their associated markers comprising:

a. running a plurality of instances of an algorithm in a distributed system wherein each instance is configured according to the following steps:

i. receiving a two-dimensional input matrix M wherein each vertical vector of the input matrix represents a single cell and each value in the vector represents a feature expression corresponding to that single cell;

ii. initialising a parent solution set $S_1, S_2, .... , S_n$ from the matrix, where n is a predefined finite number, wherein the solution set comprises a plurality of solutions $S_1, S_2, .... , S_n$ derived from the matrix, wherein each solution $S_i$ has a set of randomly assigned cluster sets $C_1, C_2, ... C_k$ of single cells, where $k$ is the total number of clusters in a given solution;

iii. computing the fitness of each solution $S_i$ in the solution set using a fitness function, wherein the fitness function is derived from a plurality of objectives;

iv. applying genetic operators to the solution set to produce an offspring solution set;

v. computing the fitness of each solution in the offspring set using the fitness function;

vi. comparing the fitness of each solution in the solution set and the offspring set, wherein each solution with the highest fitness is retained in a new solution set for the next generation;

vii. repeating steps iii, iv, v and vi for a predetermined number of generations;

b. selecting the solution set with the highest fitness of the plurality of instances;

c. extracting the single cell clusters and features from the selected solution set.

The invention also sets out a system for detecting cell types and their associated markers comprising means for executing the following steps:

a. running a plurality of instances of an algorithm in a distributed system wherein each instance is configured according to the following steps:

i. receiving a two-dimensional input matrix wherein each vertical vector of the input matrix represents a single cell and each value in the vector represents a feature expression corresponding to that single cell;

ii. initialising a parent solution set $S_1, S_2, .... , S_n$ from the matrix, where n is a predefined finite number, wherein the solution set comprises a plurality of solutions $S_1, S_2, .... , S_n$ derived from the matrix, wherein each solution $S_i$ has a set of randomly assigned cluster sets $C_1, C_2, ... C_k$ of single cells, where $n$ is the total number of clusters in a given solution;

iii. computing the fitness of each solution $S_i$ in the solution set using a fitness function, wherein the fitness function is derived from a plurality of objectives;

iv. applying genetic operators to the solution set to produce an offspring solution set;

v. computing the fitness of each solution in the offspring set using the fitness function;

vi. comparing the fitness of each solution in the solution set and the offspring set, wherein each solution with the highest fitness is retained in a new solution set for the next generation;

vii. repeating steps iii, iv, v and vi for a predetermined number of generations;

b. selecting the solution set with the highest fitness of the plurality of instances;

c. extracting the single cell clusters and features from the selected solution set.

In a preferred embodiment, the fitness function is derived from two objectives.

In a preferred embodiment, the matrix comprises raw count or $\log_2$ transcriptome or proteome data. In a preferred embodiment, the relative trajectory of a cell type is computed as the product of marker gene regulation and their correlations, wherein the trajectory of a cluster preferably defines if the regulation of a cluster is upward or downward compare to other cell types.

In a further preferred embodiment, the trajectory of each cluster $C_i$ in a solution $S_i$ is $T(C_i) =$

$$\left(\frac{expmean(C_i)}{Max(expmean(S_i))}\right) X \left(\frac{rmean(C_i)}{Max(rmean(S_i))}\right)$$

In a further preferred embodiment, the solution set selected in step (b) is selected for trajectory analysis or trajectory prediction.

In a preferred embodiment, each cluster $C_i$ within a solution $S_i$ has a variable number of gene features $G_i$.

In a preferred embodiment, each solution $S_i$ is multi-dimensional and variable in length.

In a preferred embodiment, the genetic operators comprise a crossover operator wherein the crossover operator shuffles the single cells between the clusters $C_{i...k}$.

In a preferred embodiment, the genetic operators comprise a mutation operator wherein the mutation operator replaces or adds a single cell or a feature $G_i$ within a cluster $C_i$.

In a preferred embodiment, the feature expression represents an mRNA or protein.

In a preferred embodiment, wherein the genetic operators perform adaptive operations to avoid local optima.

In a further preferred embodiment, in step vi each solution with the highest fitness is retained in a new solution set for the next generation using selection operations invoking adaptive operations to avoid local optima by removing the other solutions from the solution population $S$ and replacing them by randomly generated solutions.

In a preferred embodiment, all adaptation operations are executed by the distributed system by monitoring all instances of the algorithm.

In a preferred embodiment, the markers are regulatory markers.

In preferred embodiments, the system set out above may be adapted to carry out the method steps of the embodiments set out above.

## Brief Description of the Drawings

Embodiments of the invention are now described by way of example and with reference to the accompanying drawings in which like numerals are used to denote like parts, and in which:

Figures 1A and 1B are schematic diagrams showing different components of the distributed multi-objective genetic algorithm on high dimensional data (scaled raw count) for clustering, marker detection and trajectory analysis;

Figures 2A, 2C and 2C depict the sequential steps from sequencing to quality control (QC) (fig. 2A), the generation of Matrix M (fig. 2B) and an initial solution set $S_n$ which is randomly produced (fig. 2C);

Figure 3 shows the adaptation of running instances of the algorithm in a distributed computing setting. The adaptation node monitors the evolvability of the other x (usually x = 100) running instances of the algorithm. The adaptation node can write into the instruction file of each running instances to command the adaptation steps to maintain solution evolvability;

Figure 4 shows the adaptation of running instances of the algorithm in a distributed computing setting; and

Figures 5A-5F depict fitness plots for single cell RNAseq and protein seq data were processed for quality control (QC) and log transformed as mentioned in the algorithm step;
Clusters produced by the method according to a preferred embodiment using the best fit run for PBMC, Brain and Protein single cell data are shown in figure 6A (PBMC), figure 6B (Brain) and figure 6C (Protein).

Figure 7 shows plots for PBMC data.

Figure 8 shows plots for PBMC data.

Figures 9A and 9B show the frequency of cases where mean standard deviation in clusters of Cell Tesseract falls below (CT < random/leiden) or above (CT > random/leiden) median values of random and Leiden clusters respectively.

## Detailed Description of the Drawings

The human body is comprised of numerous types of cells. It is still not possible to identify all major cell types and their sub types. One of the major problem for disease diagnosis and treatment in precision medicine, is to identify disease associated cell types. For example, a tumor has its own cellular heterogeneity and that eventually impacts the entire body. To identify which cell in a tumor is contributing to the cancer pathogenesis, a clustering method is applied. In order to identify disease associated cell types, it is important to use biologically meaningful criteria to identify cell clusters. Traditional methods lack the use of entire data variability and reduce its dimensions to identify cell clusters. Preferred embodiments of the invention set out a method which does not reduce data variability or dimension in order to detect cell clusters in a more meaningful way by introducing meaningful biological objectives. For tumor, blood or dysplastic brain tissues in epilepsy patients, the clustering technique can be applied to decipher the cellular biological insights/targets of the disease so it can be further analysed for future drug design.

To address the problems related to single cell OMICs clustering, variable number feature selection and trajectory analysis, a method and apparatus employing a distributed adaptive multi-objective genetic algorithm is set out herein.

According to preferred embodiments of the invention, high dimension data is used without applying any data reduction method to overcome the problems related to loss of information. High dimension data retains all observable variance within the data keeping both high and low value signals which enables preferred embodiments to detect common or rare clusters from scOMICs data. Preferred embodiments of the invention apply multiple objectives to define cell clusters and their underlying gene features. These objectives are derived from molecular biology knowledge and the cell clusters are interpretable based on these defined objectives. The method and system according to the preferred embodiment is thus driven by multiple objectives and detect biologically interpretable cell types.

Motivated by the above-mentioned problems with dimensionality reduction, the method and system disclosed herein addresses them by introducing a simple metric that accelerates clustering of raw high dimensional data and with a lossless data reduction. The challenges to data clustering include loss of features during dimensionality reduction and clustering based on unknown parameters as is the case with available data clustering tools. An adaptive genetic algorithm with multiple specific objectives driven (i.e., low variance in gene regulation, high expression correlation, relative trajectory of cell clusters) data clustering, with a flexibility of adding additional objective of research interest, which can be better interpreted is disclosed herein. Preferred embodiments of the invention will identify naturally occurring cell clusters, variable number marker genes (i.e. features) associated with each cluster and the relative trajectory of a cell type that will be computed as the product of marker gene regulation and their correlations. The trajectory of the cluster defines if a cluster is upregulated in a given dataset and how consistent the cluster correlates in terms of different gene regulations i.e. the regulation is upward or downward compare to other cell types. However, single cell trajectories cannot be deduced effectively by reducing dimensionality. The trajectory is mathematically defined in equation 9.

Note that marker genes referred to as features in the models. Each cell type usually has a group of marker genes (also termed gene markers or features) that defines the cell type's unique molecular activity. The terms gene marker, marker gene and features are used synonymously herein. In a preferred embodiment, the markers are regulatory markers.

Genetic algorithm (GA) is agnostic of the dimensionality of data and can traverse through the complex exponential scale search space to produce meaningful solutions for an objective or multiple objectives. Evolutionary computation is a broad sub field within artificial intelligence that encompasses genetic programming, genetic algorithm (GA), evolutionary strategies, and other sub-branches of optimization algorithms inspired by nature. In the last decades, there has been a much research on proposing new and more effective GA, including adaptive genetic algorithm, multi objective GA and neuro evolutionary algorithm to solve optimization related problems. There have also been numerous advancements on proposing sophisticated genetic operator methods (i.e., variable length chromosome, adaptive mutation). These advancements are crucial improvements on traversing the complex search space of a problem. However, traditional algorithms are impractical to implement and to find solutions within a meaningful time. One of the most important factors for genetic algorithms is how to code precision medicine problems into its chromosome and how these coded chromosomes will evolve to produce a meaningful solution based on a fitness function that quantify the objective. Preferred embodiments address the problem of detecting cell types and their associated markers (or features) from scOMICs data sets. For genetic diseases, cell type detection is a major step forward to implement precision medicine. These cell types and their associated markers can be disease associate that can be targeted for future treatment options.

There are a lot of computational problems in precision medicine that are NP-hard and warrant approximation or heuristic based approaches to produce optimal or suboptimal solutions. Traditional diagnostic and treatment options lack molecular resolution, instead of precisely identifying only disease causing molecules, traditional approaches target a broad range of molecules and biological pathways. For example, chemotherapy lacks the ability to target causal cells for cancer, instead it targets all cells indiscriminately. One of the primary aims of precision medicine is to detect and target specific cellular molecules (i.e. cell types, features) for accelerated disease diagnosis and treatment compared to traditional approach. For example, single cell transcriptomics are at the frontier of discovering novel cell types and markers. Clustering of single cells using RNA-transcriptome data and identifying cell type specific markers is a major challenge. Each cell type usually has a group of marker genes (or features) that defines the cell type's unique molecular activity. According to the preferred method, the number of marker genes for a cell type is not fixed and each cell type can have variable number of clusters i.e. there is not fixed number on marker genes for each cluster to simplify the problem. Identifying these variable marker sets and their cell types is highly complex due to exponential search space. The similar clustering problem k-nearest neighbors is known to be NP-hard. The number of possible clusters and different features associated with each cluster is exponential and a traditional algorithm will not be able to identify a solution in a workable time. Hence, an optimization algorithm will be a better approach to find optimum or near optimum solution.

Genetic algorithms are a viable alternative for optimization related problems in high dimensional data and is impactful in accommodating multidimensionality of OMICs data such as clustering of transcriptome data. In recent years, NP-hard problem instances show genetic algorithms performing equally or even better compared to reinforcement learning. This result enforces the idea of applying genetic algorithms as a meaningful alternative to traditional algorithms and often for reinforcement machine learning algorithms.

There are critiques of GA producing suboptimal solutions due to premature convergence. However, preferred embodiments of the method described herein are configured using updated versions of genetic operators (i.e., crossover, mutation, and selection) so that they can effectively traverse search spaces and produce near optimal or optimal solutions. This requires careful design of GA including the fitness function and its relation to the problem objective. Adaptive GA are great alternative to traditional GAs and some instances of reinforcement learning algorithm. Specially for reducing the risk of stagnating at local optima, adaptive GAs can introduce needed diversity into the solution pool through the manipulation of the genetic operators. In human evolution, mutation rate varies across the genome and there are hotspots that are mutated more frequently than other regions in the genome. Following this phenomenon, an implementation of the mutation operator in GA by directing mutation into certain chromosomal region or targeting an objective yields improved results. Genetic algorithm has been previously used for feature selection on low or high dimension data.

To address the problems related to single cell OMICs clustering, feature selection and trajectory analysis, a distributed adaptive multi-objective genetic algorithm is set out herein.

The distributed adaptive multi-objective genetic algorithm according to a preferred embodiment is:

i) unsupervised clustering of single cells using high dimensional transcriptome or proteome data;

ii) identifying clusters and features (i.e., genes) based on multiple objectives on high dimensional space that is biologically interpretable;

iii) the number of clusters in a solution is not user defined, rather the algorithm of the preferred methods evolves to the best solution with a number of clusters that represents the objectives;

iv) each cluster has its own variable length feature (or gene) that defines the cluster based on the set objectives;

v) finding an optimum solution through adaptation of genetic algorithm operators (crossover, mutation, and selection) and solution set to ensure the evolvability;

vi) implementing adaptation within a cluster computing setting where each instance of the algorithm is checked for evolvability;

vii) finding the best possible solution and effective hyperparameters of genetic algorithm operators (crossover, mutation, and selection) applying distributed computing;

viii) the adaptation parameters were implemented within a distributed setting to maintain evolvability (real time) of the running instances of the algorithm;

ix) detecting single cell trajectory for each cell cluster where it was defined as the product of the marker gene regulation level and the correlation among the marker genes for each cluster on the high dimensional space.

The single cell technology is only a decade old, and methodologies are still in the development. Variants of genetic algorithms have been previously proposed mostly on low dimensional single cell data for clustering or feature (gene expression) selection. There is a huge gap for an algorithm that can handle high dimensional data from single cell OMICS for clustering and detection of their associated features that is interpretable. Single cell trajectories cannot be deduced effectively by reducing dimensionality. It is necessary to use the high dimensional values for each gene expression as it is computed from the RNA-seq. In high dimensional space, each value represents the act of numerous biological machineries and reducing dimensionality will result in losing precious biological insights from the data. For example dimensionality reduction can remove subtle variability which exists in splicing pattern but not in high dimension space. PCA cannot handle zero or extremely down regulation of genes, whereas, high dimension data will retain zeros or low mRNA seq or proteome expression data. It is also necessary to implement unsupervised clustering algorithm due to the importance of detecting subtle but important rare transcriptional dynamics (i.e. rare use of certain transcript or low level expression of certain gene markers) that might not be possible to detect using supervised machine learning approaches on low dimensional data. The preferred distributed multi-objective algorithm can detect clusters and their associated features (gene expression) from single cell transcriptome (RNA sequencing (RNA-seq)) or proteome datasets.

In the preferred embodiment, the method is a computer implemented method. Furthermore, in the preferred method, the plurality of instances are run in a plurality of computing clusters in a distributed system. Typical distributed systems configured with computing clusters may be used for the implementation. The method and system can be implemented using any suitably configured hardware and/or software means.

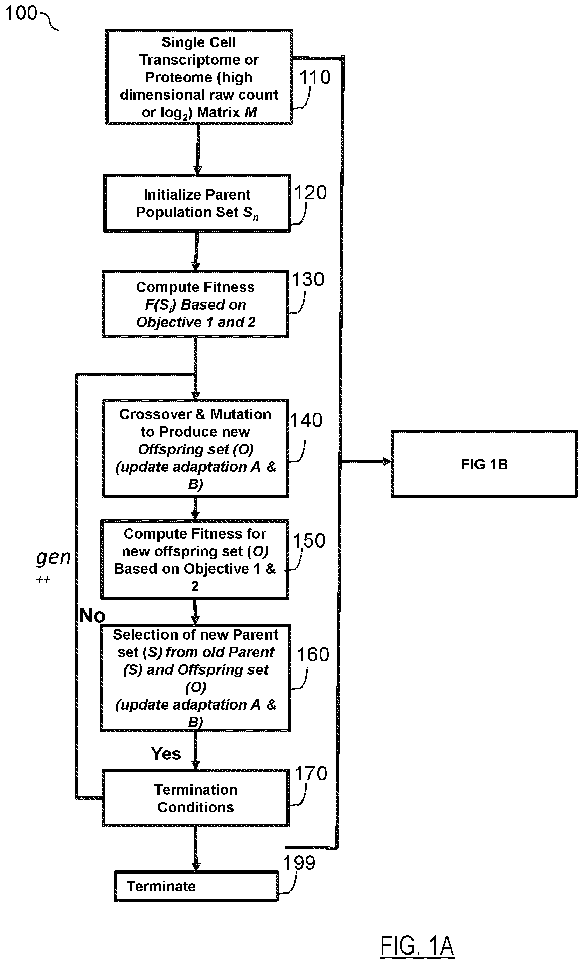

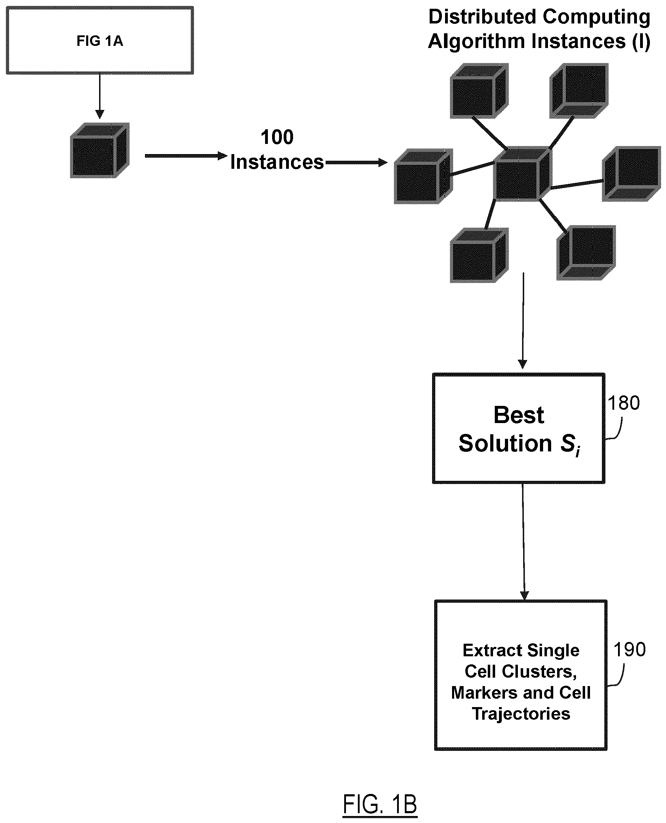

Figures 1A and 1B are schematic diagrams showing different components of an exemplary method implementing a distributed multi-objective genetic algorithm on high dimensional data (scaled raw count) for clustering, marker detection and trajectory analysis according to a preferred embodiment of the invention. The initial data usually extracted from single cell RNA or proteomics experiments where total RNA or protein from each cell of a tissue is extracted following laboratory protocols and subsequently RNA is sequenced or protein abundance is measured for thousands or millions of cells. The matrix $M$ consists of raw count or $\log_2$ transcriptome or proteome data. The algorithm initial parent solution set S and applying genetic operators (i.e. mutation, crossover) it produces offspring solution set (O). The fitness is computed for both $S$ and $O$ set and a new set of solution is selected for the next generation (gen). This process continues until the termination condition is met.

In particular, figures 1A and 1B depict the steps of an exemplary method 100 of detecting cell types and their associated markers. At step 110 a two-dimensional input matrix M is received wherein each vertical vector of the input matrix represents a single cell and each value in the vector represents a feature expression corresponding to that single cell. In the exemplary embodiment shown for illustrative purposes in figures 1A and 1B the single cell data is transcriptome or proteome, high dimensional raw count or $\log_2$. Other types of single cell data (e.g. single cell non coding RNA or metabolites) may be handled in the same manner. The invention is thus not restricted to transcriptome or proteome.

At step 120, a parent solution set $S_1, S_2, .... , S_n$ is initialised from the matrix, where n is a predefined finite number, wherein the solution set comprises a plurality of solutions $S_1, S_2, .... , S_n$ derived from the matrix, wherein each solution Si has a set of randomly assigned cluster sets $C_1, C_2, ... C_k$ of single cells, where $k$ is the total number of clusters in a given solution.

At step 130, the fitness $F(S_i)$ of each solution $S_i$ in the solution set is computed using a fitness function derived from a plurality of objectives. In this exemplary embodiment, two objectives are employed. More objectives may be employed according to circumstances.

At step 140, genetic operators (crossover, mutation, selection) are applied to the solution set to produce an offspring solution set for the next generation. In this exemplary method, crossover and mutation operators are used to produce a new Offspring set (O) and also update the adaptation parameters A and B.

At step 150, the fitness of each solution in the offspring set (O) is computed using the fitness function.

At step 160, the fitness of each solution in the solution set (S) is compared with those in the offspring set (O) and each solution with the highest fitness is retained in a new solution set for the next generation.

This process continues until the termination conditions 170 are met and the method terminates 199. This is shown in figure 1A by the loop from step 170 to 140 with an increment of the generation counter gen.

As depicted in figure 1B, the process depicted in figure 1A is run is in a distributed environment. The arrow from figure 1A to figure 1B reflects the implementation of the process of figure 1A on a plurality of distributed computing instances. In the exemplary embodiment the process as depicted in figure 1A is run on plurality of computing instances. In the exemplary embodiment 100 such computing instances are employed. More or fewer instances may be employed according to circumstances. Once the process as depicted has terminated a predetermined number of generations on each of the plurality of computing instances, the solution set ($S_i$) with the highest fitness of the plurality of instances is selected 180. The single cell clusters and features are extracted 190 from the selected solution set ($S_i$).

Methods embodying the invention, including the exemplary method disclosed herein, are typically computer implemented. In the exemplary method, the following distributed computing architecture was employed: where a number $x$ (e.g. $x = 100$) of the implemented algorithm instances are run in numerous computing clusters. The adaptation node will examine the evolvability of each algorithm instances by reading the fitness function values from each generation to measure the progress of the algorithm instance. If for any instance of the algorithm is not evolving compare to its last generation fitness values, it is because the algorithm prematurely converged to a suboptimum solution. To remove it from suboptimum solution, the adaptation node signals that instance of the algorithm to change its existing solution pool by adding new solutions so it can improve the evolvability of the algorithm.

It will be understood that other suitably configured distributed computing architecture or other hardware and/or software means may also be employed to implement embodiments of the invention.

### Quality Control of Single Cell OMICs

The average count of the raw sequencing matrix data was calculated, and features were retained if at least 10% cells have average transcript count, which were then normalized using $\log_2$ transformation. None of the single cells were excluded from analysis.

### Initializing Solution Pool S

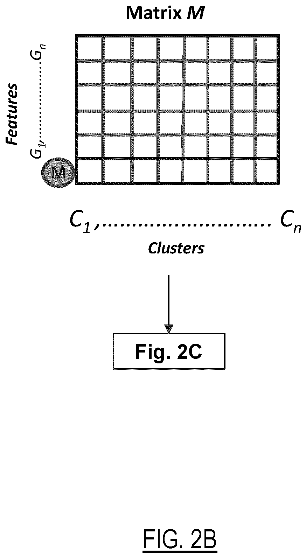

The solution space for this genetic algorithm according to a preferred embodiment is comprised of a two-dimensional matrix $M$, where each vertical vector (as shown for example in Figure 2B) represents a single cell and each value in the vector represent a feature (gene) expression (read counts obtained from mRNA sequencing or protein expression) corresponding to that single cell. In every generation, the solution population set $S$ will have $n$ solutions $S_1$, $S_2$, .... ,$S_n$ derived from the matrix $M$, where $n$ is a finite number defined by the user and usually <1000. In GA terms, these solutions are referred to as 'chromosome' and in this GA, each chromosome in the solution set $S$ has variable length of single cell clusters, and features.

Since this is an unsupervised implementation, each solution $S_i$ derived from matrix $M$ has a set of randomly assigned cluster set $C_1$, $C_2$, ... $C_k$ of single cells, where $n$ is the total number of clusters in solution $S_i$. Each $C_i$ has two variables set, $G$ - the set of $L$ features (or gene) and $R$ - the number of cells in the cluster $C_i$. The feature set in $G$ has a maximum bound of 25000 (number of genes in human genome) where each feature is a dimension in a single cell. For a $C_i$, the number of feature $L$ is drawn randomly from the set $G$. The minimum number of feature set $L$ = 25 and the maximum number of features is $L = 300$. In a large-scale single cell transcriptome study on low dimensional RNA-seq data, the number of maximum features for a cluster is 300. The number of single cells in $R$ for a $C_i$ is randomly assigned from a minimum number $min(R) = 25$ and the maximum number of single cells in a cluster bounded by $max(R) = total$ $number$ $of$ $single$ $cell$ - $((n-1) X R_{min})$.

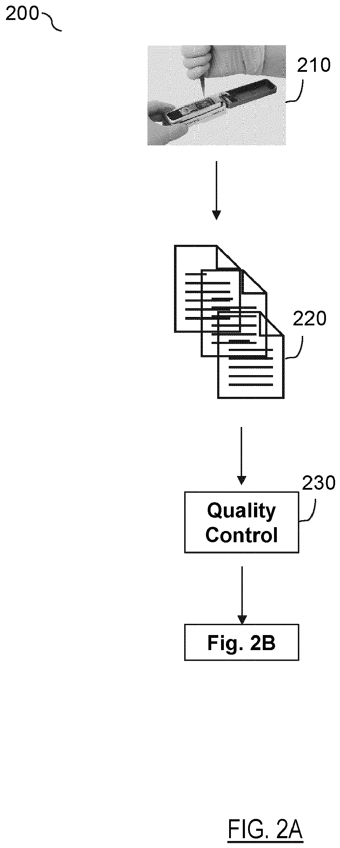

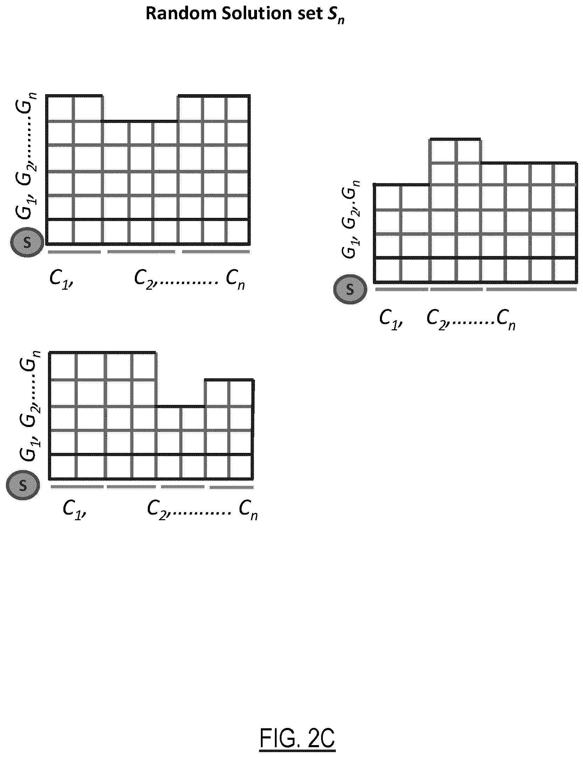

Figure 2 depicts the sequential steps from sequencing to quality control (QC) and the generation of Matrix $M$. The algorithm takes input mapped files and implement the QC before randomly producing solution set $S_n$. Each initial solution $S_i$ of the algorithm is derived from $M$ and has a random number of clusters assigned to it. Each cluster ($C_i$) has a variable number of features (or genes) ($G_i$) i.e. variable 'cluster number' and variable 'feature set ($G$)'.

In Figure 2a, step 210 depicts mRNA Sequencing or Protein Expression from lab experiments to yield single cell OMICs. The Single Cell OMICs Data are mapped and processed in step 220. Matrix M is generated at step 230 and an initial solution set $S_n$ is randomly produced at step 240.

One of the particularly advantageous features according to a preferred embodiment is the design of each solution $Si$. The solution $Si$ has a number of clusters $C_k$ assigned to it, where k is selected randomly. Each cluster $C_i$ within a solution $S_i$ have a variable number of gene features ($G_i$) (see figure 2C). Hence, each cluster within a solution set can have different number of independent features. To have biologically meaningful features for a cluster, the length |G| of the features should have a user defined minimum (e.g. 20 or 25). The cell type with a fixed gene feature approach may not allow some of the important marker to get selected due to the restriction. This variable number gene features will allow to detect cell types with right number of marker genes and will increase the precision of cell clustering. Hence, for cell cluster $C_1$ the length of |G| might be 20, whereas for $C_2$ there may contain 25 gene features. This ensures that the single cell clusters and features extracted from the selected solution set are biologically interpretable. In particular, the extracted single cell clusters and features are biological interpretable by having right number of marker genes rather than applying dimension reduction. Figures 2A, 2B and 2C thus reflect how, for example, mRNA Sequencing or Protein Expression takes place in lab experiments and the resultant input data is processed by the automated method/system according to the preferred embodiments of the invention to yield single cell OMICs. As the resultant extracted single cell clusters and features are biologically interpretable medical diagnosis and therapeutics are facilitated.

Furthermore, data variability is not reduced by the methods set out in the preferred embodiments rather preserved as it is. Single cell OMICs variability is intrinsic and multi dimensional. The data variability exists due to the active involvement of numerous biological process involving cell molecules and traditional clustering algorithm just produces clusters based on underlying variance from a reduction technique without interpolating any biological knowledge into it. The methods set out in the preferred embodiments use the high dimension data from RNA sequencing and the algorithm implements specific objectives to cluster the scOMICs data. For example, if we set an objective applying biological knowledge and want to observe clustering of single cell OMICs regarding how similarly the cells genes are expressed, the method according to preferred embodiments clusters scOMICs data based on this particular objective.

## Compute Fitness

For each solution $S_i$, fitness is computed in two layers. First, assign each cluster $C_i$ a fitness function that was derived from two primary objectives. Second, a final fitness was derived for each solution $S_i$ computed from the fitness values assigned to its cluster set $C_{i,..k}$. The fitness function is essentially a minimization function where both objectives will have low variance (distance) for a cluster with closely linked single cells. In preferred embodiments, the two objectives are both very important for real world applications, especially to identify cell types that molecularly are behaving in a similar way. For example, an objective can be derived where cell types will be clustered based on cell type. However, in embodiments the objectives are more into molecular similarity.

_Objective 1_: The first objective in the fitness function defines the average correlation of each single cell computed using equation 1, 2, and 3, where the feature vectors of the single cells within a cluster $C_i$ was used to compute correlation co-efficient $r$. This objective computes the mean $r$ for a cluster $Ci,rmean$.

Average correlation $r$ for each single cell in a cluster $C$ with $R$ single cell and $L$ features in set $G_i$ was computed as:

$$r(C_i) = \frac{\sum_{a=1}^{R-1} Corr\ (C_{i},C_p)}{R-1}\ , \text{ when } i \neq p \tag{1}$$

$$Corr\ (C_i, C_p) = \frac{(L*\sum_{a=1}^{L}(G_{i,a}-G_{p,a}))-(\sum_{a=1}^{L}G_{i,a}*\sum_{a=1}^{L}G_{p,a})}{\sqrt{((L*\sum_{a=1}^{L}(G_{i,a}*G_{i,a}))-(\sum_{a=1}^{L}G_{i,a}*\sum_{a=1}^{L}G_{i,a})))* ((L*\sum_{a=1}^{L}(G_{p,a}*G_{p,a}))-(\sum_{a=1}^{L}G_{p,a}*\sum_{a=1}^{L}G_{p,a})))}}$$

$$\tag{2}$$

$$\text{Mean Correlation of a cluster } rmean(C_i) = \frac{\sum_{a=1}^{R} r(C_i)}{R} \tag{3}$$

*Objective 2*: The second objective is to compute the mean expression *'exp'* of each single cell and its relation to the cluster. This objective computes (equation 4 and 5) the mean expression of a cluster *expmean(Ci)*.

$$\text{Expression difference } exp(C_i) = \frac{\sum_{a=1}^{L} absolute\ (G_{i,k,}-G_{i,a})}{L} \tag{4}$$

$$\text{Mean expression of a cluster } expmean(C_i) = \frac{\sum_{i=0}^{R} exp(C_i)}{R} \tag{5}$$

The fitness of a cluster $F(C_i)$ was assigned by dividing the maximum possible distance computed (equation 6 and 7) for the value (0,0) and (1, max feature expression (raw read count or $log_2$)). Next, the solution fitness $F(S_i)$ is the average fitness of all the cluster fitness (equation 8). Maximum the fitness is, the more chance it has to get selected for the breeding offspring. The maximum fitness also has the inverse relation with the distance function derived from objective 1 and 2. In other words, the higher the fitness of a solution means lower the distance between a single cell and its cluster mean for objective 1 and 2.

$$\text{Distance between two points } d(C_i) = \sqrt{(exp(c_i) - expmean(C_i))^2 - (r(C_i) - rmean(C_i))^2} \tag{6}$$

$$\text{Cluster Fitness } F(C_i) = \frac{maximum\ distance\ D}{\sum_{i=0}^{R} d(C_i)} \tag{7}$$

$$\text{Solution fitness } F(S_i) = \frac{\sum_{i=0}^{n} F(C_i)}{n} \tag{8}$$

The trajectory T of a cell cluster was computed based on (equation 9), the two defined objectives and it tracks the state of gene expression and their correlation of the single cells in a cluster. Trajectory of each cluster $C_i$ in a solution $S_i$ is

$$T(C_i) = \left(\frac{expmean(C_i)}{Max(expmean(S_i))}\right) X \left(\frac{rmean(C_i)}{Max(rmean(S_i))}\right) \tag{9}$$

Preferred embodiments accommodate two objectives. The illustrative embodiments set out herein are set out with two objectives; however, in other embodiments, additional objectives are accommodated as well. There can numerous types of objective that can be added and implemented according to such embodiments. For example: 1) cluster cell types based on dis-regulation of a set of disease associated marker gene (or features) or 2) cluster cell types based on only upregulated marker genes or down regulated marker genes.

**Selection of Parents for New Offspring**

An elitist method has been implemented to retain the most fit solutions for the next generation. Both parent and offspring with equal number of solutions in sets *parent(S)* and *Offspring(O)*, respectively, compete to make it into the new solution set. Fitness of each $S_i$ and $O_i$ from the parent and offspring set was ranked and the top $n = |S|$ set with the highest fitness was retained for next generation breeding. To select the next generation, the offsprings (from crossover and mutation) and the parents compete and the top $n$ solutions make it to the next generation of $S$.

The adaptation parameter A in a preferred embodiment will check if the fitness function converged after a certain (user defined) number of generations *gen* (in this exemplary embodiment *gen = 100*). If the fluctuation of average fitness is 0 within these previous few generations, the algorithm will update adaptation parameter A = 1 (default is 0). If A = 1, the algorithm will take measures to adopt to remove the existing solutions from S to move the search from premature convergence position within the search space (see adaptation section for details). The algorithm will only keep the best solution, *best(S_i)* with highest fitness in the parent, *parent(S)* set and all other $S_i$ will be replaced by a random set of $n$-1 solutions and will subsequently be used to produce another $n$ offspring for the set *Offspring(O)*. If the adaptation *A = 0*, the algorithm will move onto next step without replacing any solution in set *parent(S)*. The application of adaptation parameter accelerates the evolution of the algorithm and moves the algorithm away from producing sub optimal solutions.

**Crossover & Mutation Operator**

Two selection processes have been implemented for crossover operation. Each solution $S_i$ is multidimensional and variable in length. Due to this complexity, the crossover operator shuffles the single cell between the clusters $C_i$. Two selection criteria are applied to shuffle the single cells between the clusters $C_i...k$. First selection will pick a random solution $S_i$ and will randomly pick two cell clusters within $S_i$. In the next step, the single cells between the clusters will be swapped without swapping their associated features $G$.

The second selection criteria picks one of the top (highest fitness) five solutions $S_i$ and the cluster that has the weakest fitness will be swapped to a cluster that has the closest mean fitness. Although it is a probabilistic approach, it is more direct and impactful. The probability of using one of the two selections in each generation is equal ($p = 0.50$). If the algorithm detects a premature convergence, and the adaptation parameter A = 1, the probability of using the second selection criteria for crossover will be increased by 80% or (probability will be assigned, $p = 0.80$).

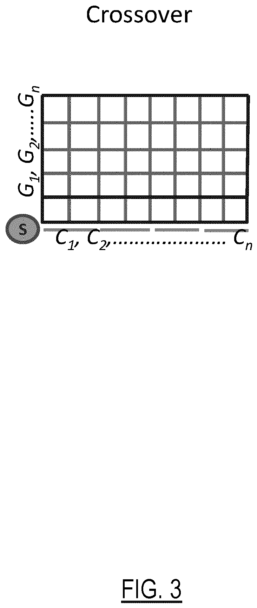

The exemplary crossover operation depicted in figure 3 operates as follows: select a random solution S from the solution pool. Distance between each $Ci,j$ and the clusters mean Corr and mean Exp compared. $Ci,j$ is placed to the closest C and it adopts the G that belong to the new C. The crossover also have adaptation switch. If A is on, instead of a random solution S, it will put more probability of picking the fittest solution within the pool. Looking back to the schematic diagram in figure 1A, crossover and mutation are used after computing fitness. These operations are used to produce next generation solution.

The mutation operator was designed to (randomly) replace or add a single cell or a feature $G_i$ within a cluster $C_i$. First, for single cell mutation operation, the method is configured with a directed approach where selection will pick a cluster $C_i$ randomly from a solution $S_i$ and only selects deterministically the weakest (lowest fitness) single cell within that cluster to replace. The weakest single cell of $C_i$ will be replaced by the weakest single cell of another cluster $C_p$, where $i \neq p$ and $C_p$ is less distant to this particular single cell. No delete operation is conducted on single cell due to the importance of each single cell of the data.

Secondly, the mutation operation randomly deletes, or adds a feature from a feature $G_i$ within a cluster $C_i$. The operation includes deletion of an existing feature and adding a new feature into $G_i$, randomly drawn from 25000 features in the genome. If the algorithm detects a premature convergence, and turns A = 1, the mutation rate increases by 50%, to introduce more diversity into the solution set S.

**Distributed Computing and Adaptation to Ensure Evolvability of Solutions:**

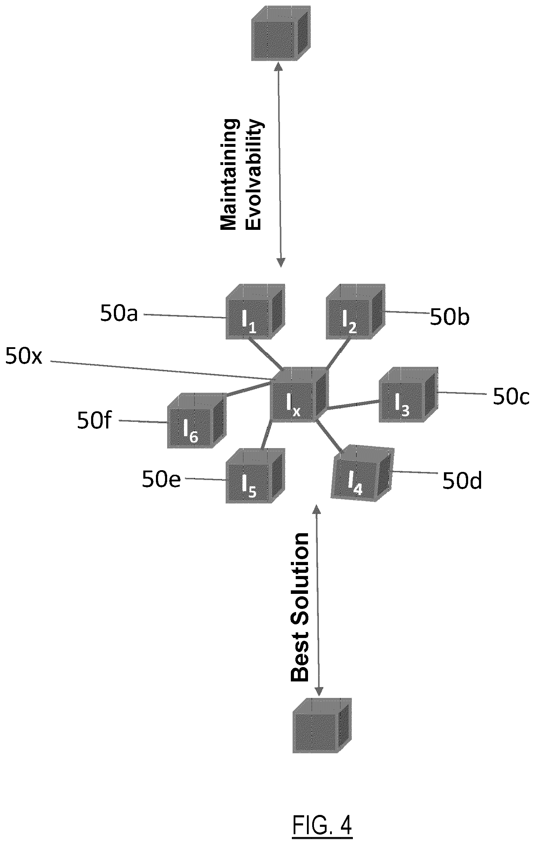

The algorithm is comprised of several parameters (mutation rate, number of generations (*gen*), selection probability) and it is hard to run a test set to find the optimum values for these parameters. The exemplary method and system are preferably implemented using a distributed computing approach. According to an exemplary embodiment, 100 instances of the algorithm run with assigning different genetic algorithm parameter values in each instance. According to a preferred embodiment, all instances of the algorithm are monitored. The algorithm updates adaptation variable A or B for a set of parameter values in a single running instance *I*. The 'best solution' will be determined by comparing fitness value of each best solution produced by each instance of the genetic algorithm. This 'best solution' is used for obtaining results related to clustering, feature detection and trajectory analysis (Figure 4). According to an exemplary embodiment, the method is configured using the specific steps below for adaption within a distributed running environment:

- The adaptation node within the distributed computing architecture (Figure 4) initiate $x$ number of running ($x$ = 100 in the exemplary embodiment, though $x$ can be adjusted according to preferences) instances $I_x$ of the algorithm and each running instance is monitored by the adaptation node to ensure if the fitness is evolving according to the specified objectives or not. The node can send real time instruction to a running instance of the algorithm to change its internal parameters or stop that run and replace it by a new instance.
- To enhance evolvability, the adaptation node parameter A *(by default set to 0)* checks every $x$ generation for the convergence of average fitness. It the adaptation node finds a premature fitness stagnation point for an instance of a program it sets $A$ = 1 (instruction file). It $A$ = 1, that particular instance of the program removes all solutions from set $S$ except retaining the best fit solution. The rest of the solutions were randomly generated for the next generation to compete. Hence, the next generation solution will have the exact number of solutions like before and will include only the best of parents and offsprings.
- Due to poor evolvability (no fitness progression) if termination parameter for an instance of the program is $B$ = 1 (by *default set to 0),* adaptation node stops this program and restarts a new instance of the program by increasing mutation rate, with a new population. At the end, the adaptation node will have $x$ = 100 runs.

Figure 4 shows the adaptation of running instances of the algorithm in a distributed computing setting. The adaptation node monitors the evolvability of the other $x$ ($x$ = *100* in the exemplary embodiment, though $x$ can be adjusted according to preferences) running instances of the algorithm. The adaptation node can write into the instruction file of each running instances to command the adaptation steps to maintain solution evolvability.

**Single Cell Datasets:**

Dataset 1: 2,700 single cells from PBMC (peripheral blood mononuclear cell). The raw RNAseq data were obtained from (https://satijalab.org/seurat/archive/v3.1/pbmc3k tutorial.html).

Dataset 2: 7,283 single cells from ACC (Anterior Cingulate Cortex) region of brain. The raw RNAseq data were obtained from Allen Brain Atlas (https://portal.brain-map.org/atlases-and-data/rnaseg)

Dataset 3: Single cells from CD8+ naive T cells. The raw protein sequencing assay data were obtained from GSE100501 (https://www.ncbi.nlm.nih.gov/geo/guery/acc.cgi?acc=GSM268S2S8).

These datasets have been used in prior art PCA based approaches. Using these datasets enables comparison against such prior art approaches.
For comparison purposes, a few clusters were randomly created and assigned randomly single cells into those clusters with random number of features in each cluster.

**Comparison Tests:**

The Cell Tesseract (CT) is an exemplary implemented version of the distributed adaptive multi-objective genetic algorithm used in preferred embodiments of the invention. Analysis has been conducted using the most frequently used single cell transcriptome software as described in Seurat which implements the unsupervised cluster algorithm 'Leiden' that implements a network-based algorithm that partition data based on their network membership. In both comparisons, the dispersal (standard deviation (stdev)) of feature (gene) expression of a cluster identified by Cell Tesseract is compared with the distribution of stdev of features in clusters that were randomly assigned or detected by Leiden algorithm. One proportion Z-test may be used to estimate the significance.

**Results**

**Clustering of single cell data using Cell Tesseract**

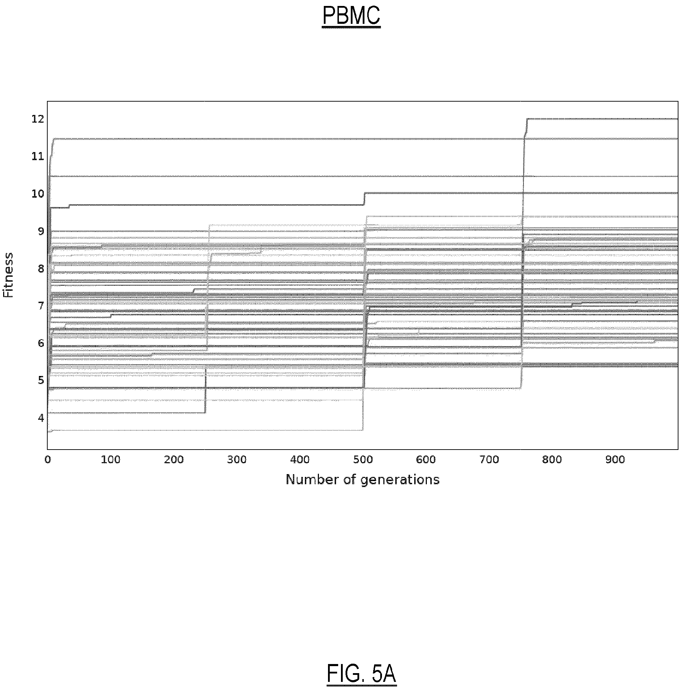

Single cell RNAseq and protein seq data were processed for quality control (QC) and log transformed as mentioned in the algorithm step. Each dataset was run with different set of parameters to identify the best solution $S_j$. The fitness of each run was monitored across generations to confirm that the algorithm is evolving within the search high dimensional space. The results are shown in figures 5A-5F.

Figure 5A shows the fitness plot for PBMC (1000 generation, 60 runs).

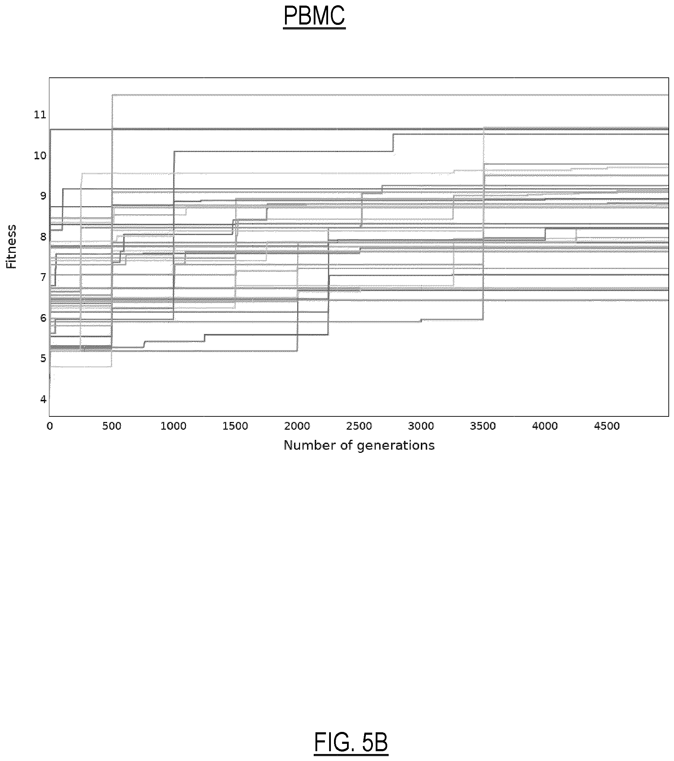

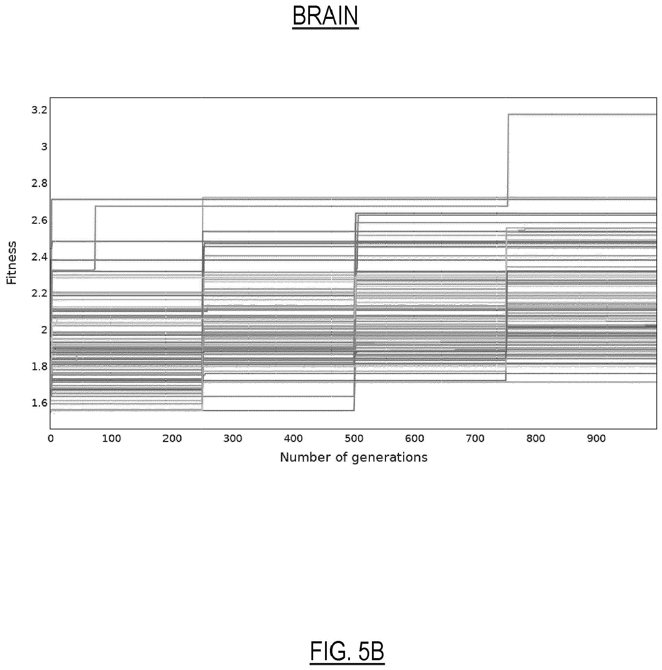

Figure 5B shows the fitness plot for PBMC (5000 generation, 40 runs).

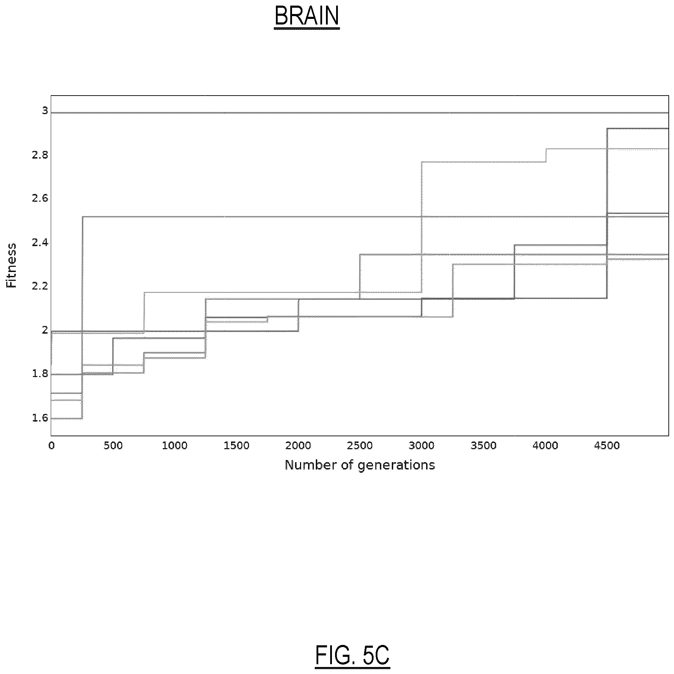

Figure 5C shows the fitness plot for Brain (1000 generation, 93 runs).

Figure 5D shows the fitness plot for Brain (5000 generation, 7 runs).

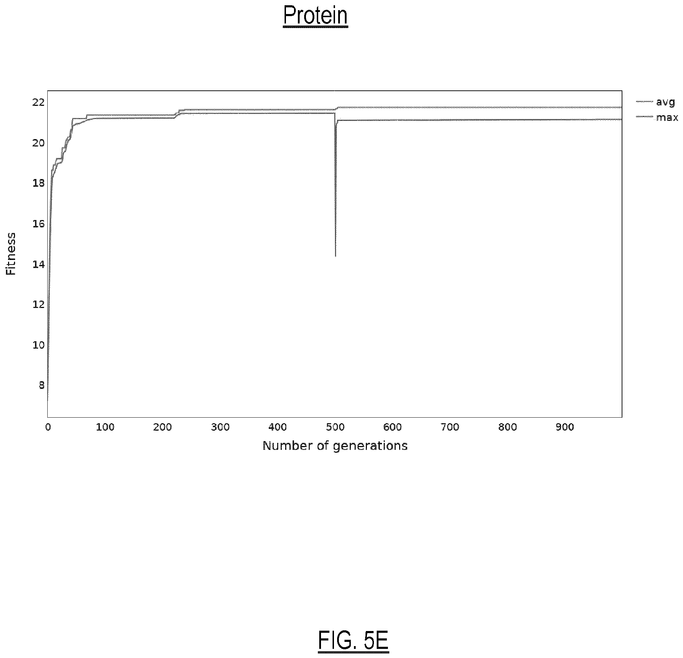

Figure 5E shows the fitness plot for Protein single cell data (1000 generation, 1 run),

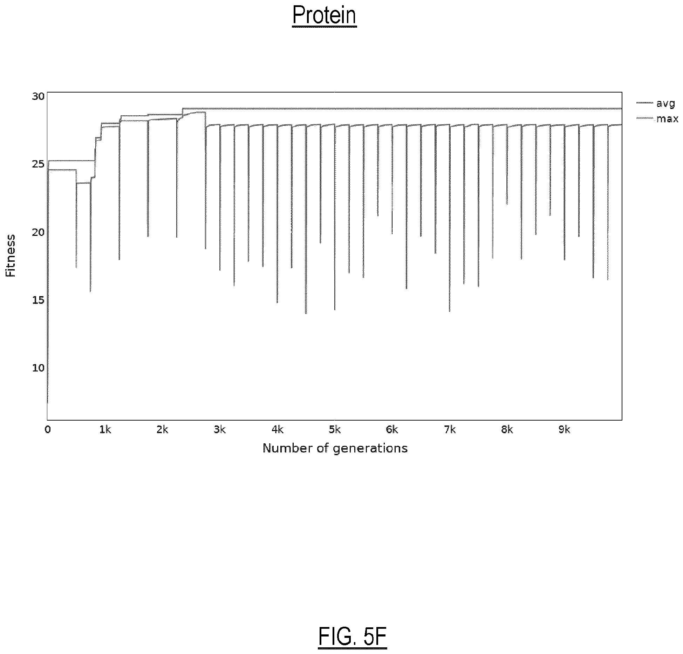

Figure 5F shows the fitness plot for Protein single cell data (10000 generation, 1 run). The fitness of each run is represented in different colors, which can be recognised as different shades in the black and white rendering.

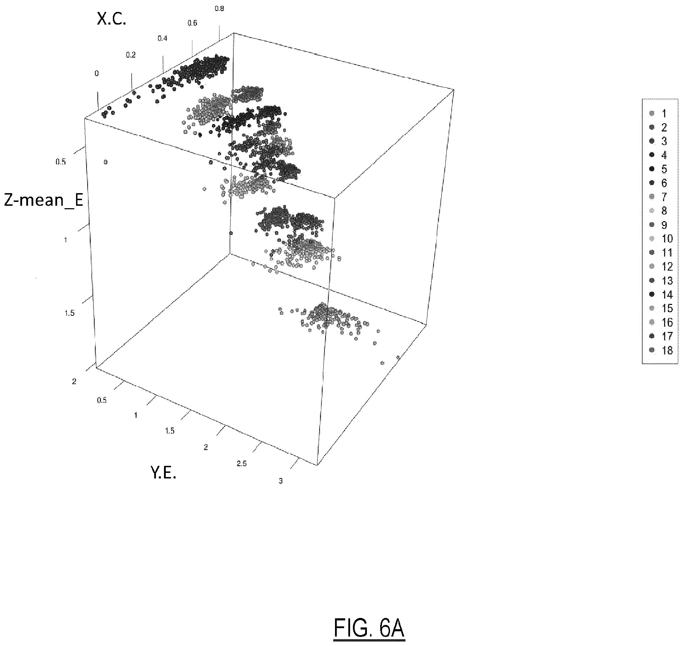

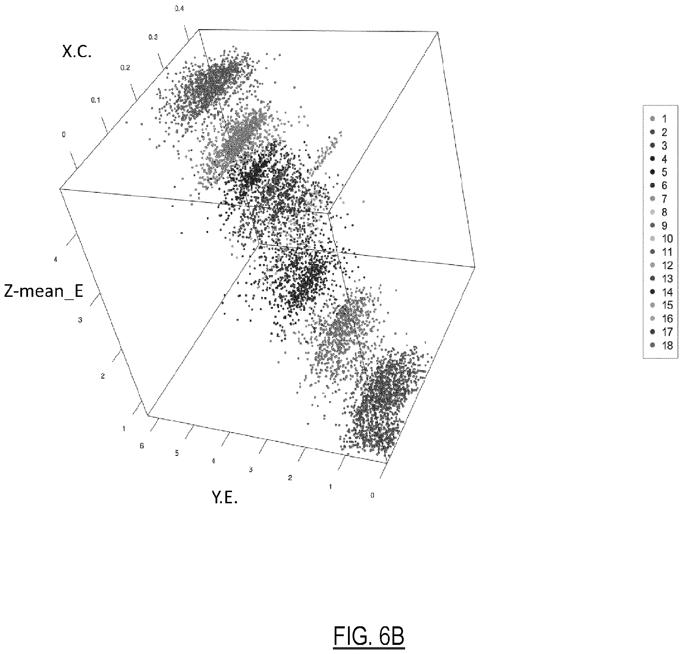

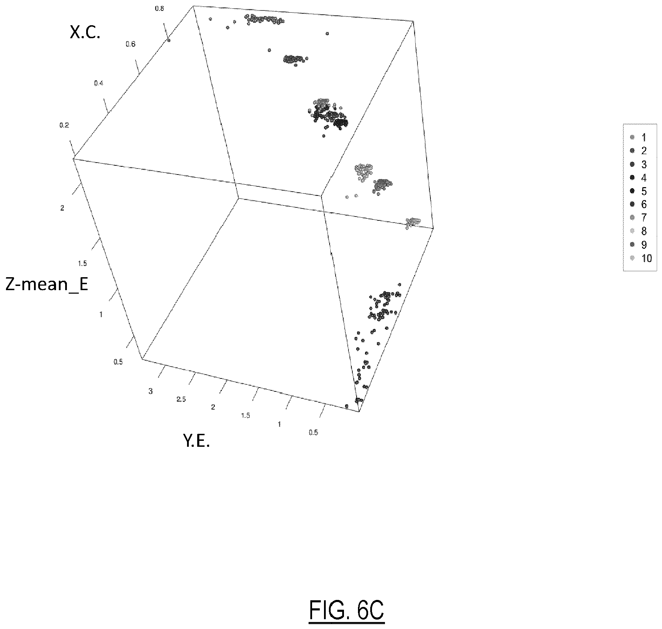

The evolvability of the algorithm runs show multiple fold increase of the fitness from the starting point. In an exemplary embodiment, the results from 100 runs of PBMC single cell data for comparison purposes. From distributed runs, the best solution with highest fitness was chosen for data clustering, feature selection and trajectory prediction. Clusters produced by the method according to a preferred embodiment using the best fit run for PBMC, Brain and Protein single cell data are shown in figure 6A (PBMC), figure 6B (Brain) and figure 6C (Protein).

The clusters are visualized using plotly of Rgl package in R, individual clusters are numbered and represented by different colors, which can be recognised as different shades in the black and white rendering. XC, YE and Zmean E denotes correlation, average mean expression, and the trajectory T respectively. Trajectory Tin Z-axis is a product of mRNA expression level and the correlation of marker genes for a cluster. Each cell type defines its unique trajectory due to variable number marker gene. The trajectory $T$ is defined mathematically in equation (9).

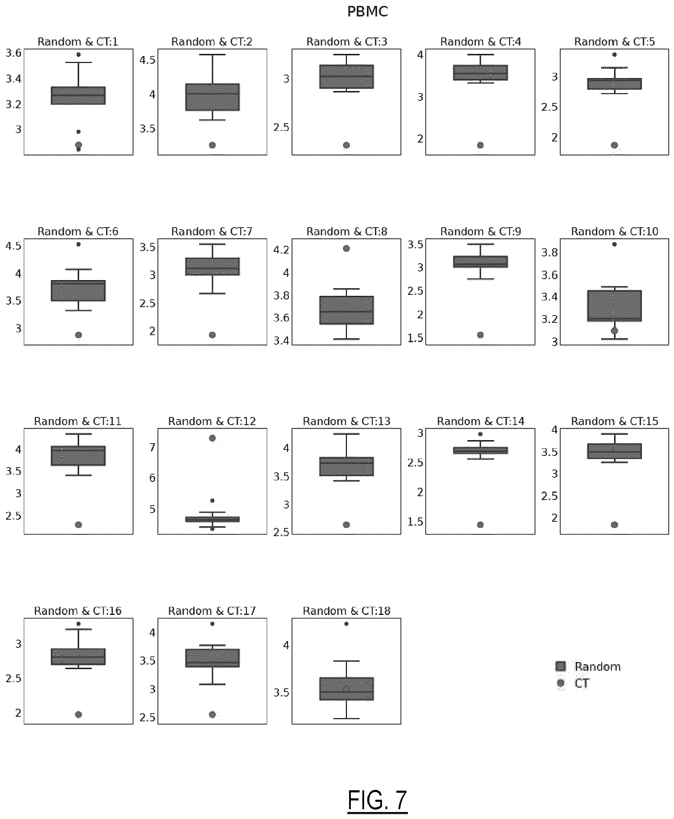

To test the performance of Cell Tesseract algorithm as employed in a preferred embodiment, the mean standard deviation (stdev) of features (gene expression) in individual clusters generated by Cell Tesseract is compared to the respective distribution of standard deviation in all clusters generated by a random cluster assignment. This allows us to compare each cluster's standard deviation of CT with the distribution of standard deviation from randomly generated clusters and their associated features. The plots for PBMC data are shown in Figure 7.

The mean standard deviation (y-axis) calculated for the features in Cell Tesseract (CT) (red dot) individual clusters (CT1 to CT18) in comparison to all clusters using Random cluster assignment (distribution shown as box plot) using PBMC data. The result shows the mean and its deviation of Cell Tesseract is significantly different than randomly generate results.

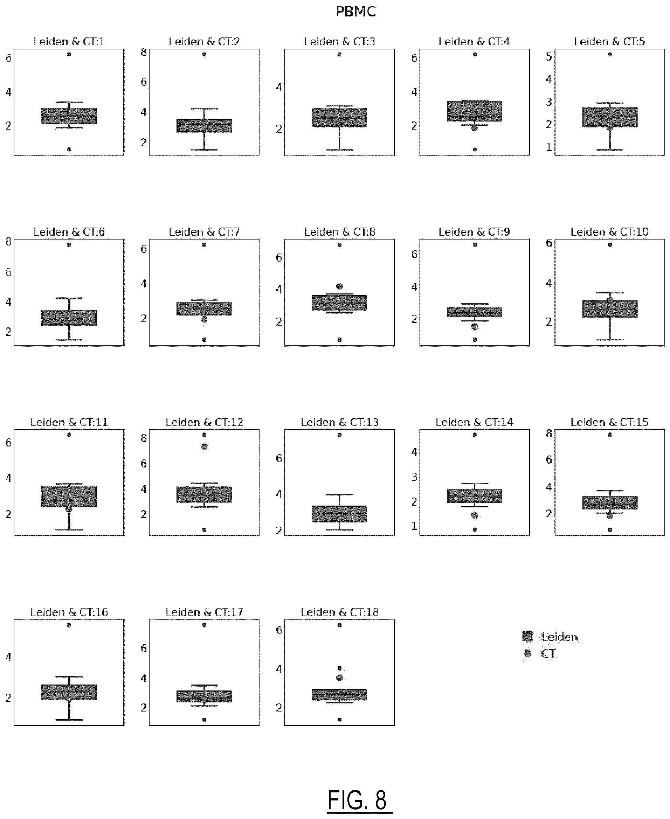

Further, the mean standard deviation of features in individual clusters generated by Cell Tesseract is compared to the respective distribution of standard deviation in all clusters generated by one of the gold standard clustering algorithms (in low dimensional space), Leiden (Figure 8). The results show that standard deviation of Cell Tesseract cluster features are not significantly different compared to Leiden. Next, the outcome of both comparison (random assigned clusters and Leiden) is quantified. The results show that overall Cell Tesseract clusters show significantly ($P < 7.3 \times 10^{-5}$) less standard deviation of cluster expressions compared to randomly assigned clusters, whereas Cell Tesseract is not significantly ($P < 0.16$) different compared to Leiden (Figure 8).

Figure 8 show the mean standard deviation (y-axis) of features in Cell Tesseract (red dot) of individual clusters (CT1 to CT18) in comparison to all clusters using Leiden algorithm (distribution shown as box plot) using PBMC data.

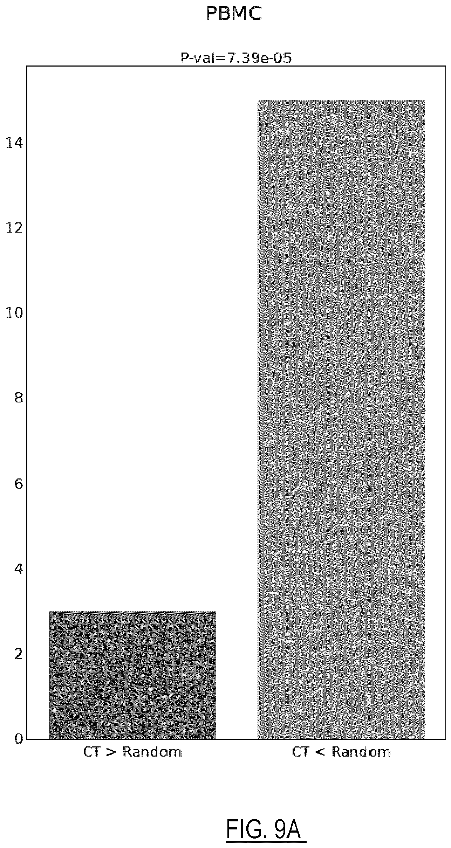

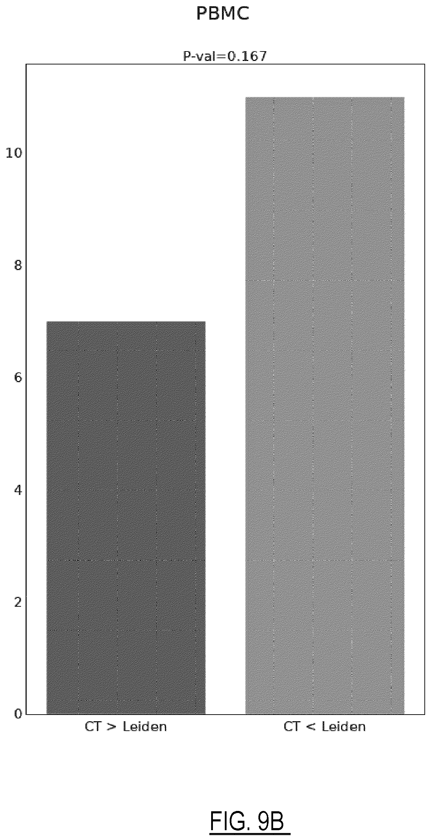

Figures 9A and 9B show the frequency of cases where mean standard deviation in clusters of Cell Tesseract falls below (CT < random/leiden) or above (CT > random/leiden) median values of random and Leiden clusters respectively. The mean standard deviation of CT is significantly different compare to randomly generated cell clusters and features; whereas; the mean standard deviation is similar to Leiden clustering algorithm. Hence, the method according to the preferred embodiment is performing significantly better than randomly generated results and it is comparable to the commonly used Leiden clustering algorithm.

**Discussion**

The method according to preferred embodiments is configured using a genetic algorithm that can handle both single cell transcriptome and proteome data for clustering considering biologically driven objectives. The implemented objectives enable the detection of cell types where the level of marker genes are similarly regulated (i.e. similar mRNA-expression level) and the correlation of mRNA-expression between the cells are high. The objectives also enable the identification of the trajectory of each cell type, meaning, the regulation is upward or downward compare to other cell types. The method according to such a preferred embodiment searches for an optimum solution within high dimensional single cell OMICs data. In an exemplary implementation, it has been applied to both transcriptome and proteome data to obtain objective driven clusters that are interpretable according to the objective. This preferred method can thus be utilized to identify disease causal cell types and their associated marker genes. The first objective in the exemplary method investigates the correlation between the single cells in a cluster. The second objective quantitate the level of coexpression dynamics of single cells within a cluster. Both objectives are later used to identify the trajectory of the cluster that defines if a cluster is upregulated in a given dataset and how consistent the cluster correlates in terms of different gene regulations. The number of clusters and the number of features in a cluster is unknown. This unsupervised nature of searching for the optimum solution make it flexible to identify clusters with different quantitative measurements.

Since no dimensionality reduction technique is applied, the exemplary method is configure to handle thousands of dimensions to find objective driven interpretable clusters from single cell OMICs data. Although high dimensionality of the data introduces enormous complexities, Cell Tesseract, the exemplary implementation of the preferred method, evolves to find its way to the biologically meaningful solutions. Each solution represents a set of cell clusters where their marker genes are similarly regulated in terms of mRNA expression level and regulation correlation. Unlike traditional dimension reduction methods, the preferred method provides this information that helps interpret the cluster with meaningful molecular insight. The clusters have been validated, comparing with randomly assigned cluster and the most frequently used single cell clustering algorithm in Seurat. The exemplary method according to a preferred embodiment of the invention significantly ($p < 7.3X\ 10^{-5}$) outperformed random cluster assignment showing the clusters comprised of highly correlated single cells and detects feature sets that co-express similarly. The resultant data also produces comparable ($p < 0.16$) results in terms of mean standard deviation against Seurat. A preferred method that introduces variable solution or chromosome length, adaptation parameter, and directed genetic operators to find clusters, features, and trajectories from single cell OMICs dataset is thus set out herein.

The method and system configured using the exemplary genetic algorithm set out herein provide a solution for single cell clustering algorithm that efficiently can detect clusters and their associated variable number of features for OMICs (i.e. transcriptome or proteomics) dataset. This GA works on high dimension data that preserves the observable variation within the OMICs molecules. The exemplary GA applies objective driven clustering that is interpretable in light of the set objectives and can be applied to numerous single cell OMICs data, including conditional (treated or not treated) data. For example, the preferred method can be applied to case control scOMICs data with the objective to find disease causal cell types in cases. From patient and control tissue the algorithm is capable to identify the cell types and their marker genes that are regulated differently in patient tissue not in controls (i.e. triple negative breast cancer). The preferred method can also be applied to single cell proteomics data obtain from patient tissue to identify protein markers for a disease (e.g. epilepsy). The GA described in the context of the preferred embodiment thus improves on PCA based two dimensional clustering as shown in the results.

The preferred embodiments thereby avoid creating undesirable linear correlation between variables which may lead to failure in situations where datasets cannot be defined by the mean and covariance. Also, low dimensional approaches must neglect certain variables during feature selection. The high dimensionality approach set out in the preferred embodiments overcomes the problem of selecting features which depend on univariate statistics and may lead to suboptimal subset.

Moreover, the high dimensionality approach set out in the preferred embodiments overcomes the problem of bias towards high value signals (e.g., high expressed feature cluster). Instead, the preferred embodiments capture low signals from the data (i.e., small rare low expressed cell clusters in scRNA-seq or other OMICs data).

One of the limitations found in Scanpy output is that cells of the same type aren't always grouped together, and cells of different types aren't well separated. One of the general concerns for supervised clustering methods such as k-means is that user must provide number of clusters or the resolution which determines the number of clusters. These complexities highlight the need for a better unsupervised solution to detect clusters and their associated features (i.e. genes).

**Specific applications of the preferred embodiments**

As stated above, single cell is a very new technology that is only a decade old. The methods and systems according to preferred embodiments can impact the diagnostics and therapeutics of many genetic diseases that are currently not druggable with traditional approaches. Below is a list of high stake applications of the preferred embodiments:

1. The methods and systems according to preferred embodiments identify cell specific markers using high dimensional single cell OMICs (scOMICs) data (e.g. transcriptome or proteome). These markers that belong to a specific cell type are the primary regulator for a group of cells. Marker identified from our algorithm can be tested as a diagnostic predictor for a disease. Such markers can be applied to clinical trials to quantify the diagnostic sensitivity and specificity. Such markers can be used widely, and this approach can be applicable to various diseases. For example, currently there is no marker for most of the rare genetic diseases and cancers. The methods and systems according to preferred embodiments can be applied to such undruggable diseases to identify the scOMICs based disease markers and their associated cell types. Diseases to which the preferred embodiments can be readily applied are epilepsy and breast cancer. In both cases the preferred methods and systems can be applied to find predictive markers for diagnosis and therapeutics.

2. The methods and systems according to preferred embodiments can be applied to gene therapy. For example:

> a. CRISPR/Cas9 can be designed in a cell type specific manner and methods and systems according to preferred embodiments can identify such cell types and target molecules for CRISPR/Cas9 design.

b. Antisense oligonucleotide (ASO) based mRNA drug technology: methods and systems according to preferred embodiments can target cell type specific markers that are upregulated in a genetic disease. The marker detection will be the key for targeting the cell with ASO mRNA drug. For example, if we apply scOMICs using breast cancer tissues, methods and systems according to preferred embodiments will be able to detect cell types and their associated markers that are primary regulator of the phenotype. ASO mRNA technology can then be applied to target these markers to block or reverse the regulation. This implementation can be applied widely to most genetic diseases.

3. Finding out cell type specific markers can bring huge opportunity to collaborate with pharmaceuticals. Recently drug design industry adopting single cell technology for single cell level molecule quantification. Methods and systems according to preferred embodiments can be applied to treated and untreated type of pharmacogenetic testing experiments in a cellular model for various drug compounds to identify marker sensitivity and specificity against a chemical compound or drug.
4. As can be seen above, the methods and systems according to preferred embodiments can be applied widely. Research collaboration with numerous genetic and cell biology research groups is thus an obvious opportunity. Additional predictive functionality may be added for different biological questions involving single cell OMICs.

The invention is not limited to the embodiments described herein but can be amended or modified without departing from the scope of the present invention as defined by the appended claims.

**Claims**

**1.** A method of detecting cell types and their associated markers, the method comprising:

a. running a plurality of instances of an algorithm in a distributed system wherein each instance is configured according to the following steps:

i. receiving a two-dimensional input matrix M wherein each vertical vector of the input matrix represents a single cell and each value in the vector represents a feature expression corresponding to that single cell;
ii. initialising a parent solution set $S_1, S_2, .... , S_n$ from the matrix, where n is a predefined finite number, wherein the solution set comprises a plurality of solutions $S_1, S_2, .... , S_n$ derived from the matrix, wherein each solution $S_i$ has a set of randomly assigned cluster sets $C_1, C_2, ... C_k$ of single cells, where k is the total number of clusters in a given solution;
iii. computing the fitness of each solution $S_i$ in the solution set using a fitness function, wherein the fitness function is derived from a plurality of objectives;
iv. applying genetic operators to the solution set to produce an offspring solution set;
v. computing the fitness of each solution in the offspring set using the fitness function;
vi. comparing the fitness of each solution in the solution set and the offspring set, wherein each solution with the highest fitness is retained in a new solution set for the next generation;
vii. repeating steps iii, iv, v and vi for a predetermined number of generations;

b. selecting the solution set with the highest fitness of the plurality of instances;
c. extracting the single cell clusters and features from the selected solution set.

**2.** The method of claim 1, wherein the fitness function is derived from two objectives.

**3.** The method any of claims 1 or 2, wherein the matrix comprises raw count or $log_2$ transcriptome or proteome data.

**4.** The method of any preceding claim, wherein the relative trajectory of a cell type is computed as the product of marker gene regulation and their correlations.

**5.** The method of claim 4 wherein the trajectory of a cluster defines if the regulation of a cluster is upward or downward compare to other cell types

**6.** The method of any of claims 4 or 5 wherein the trajectory of each cluster $C_i$ in a solution $S_i$ is

$$T(C_i) = \left(\frac{expmean(C_i)}{Max(expmean(S_i))}\right) X \left(\frac{rmean(C_i)}{Max(rmean(S_i))}\right)$$

7. The method of claim 4, wherein the solution set selected in step (b) is selected for trajectory analysis or trajectory prediction.

8. The method of any preceding claim wherein each cluster $C_i$ within a solution $S_i$ has a variable number of gene features $G_i$.

9. The method of any preceding claim wherein each solution $S_i$ is multi-dimensional and variable in length.

10. The method of any preceding claim wherein the genetic operators comprise a crossover operator wherein the crossover operator shuffles the single cells between the clusters $C_{i...k}$.

11. The method of any preceding claim wherein the genetic operators comprise a mutation operator wherein the mutation operator replaces or adds a single cell or a feature $G_i$ within a cluster $C_i$.

12. The method of any preceding claim wherein the feature expression represents an mRNA or protein.

13. The method of any preceding claim wherein the genetic operators perform adaptive operations to avoid local optima.

14. The method of claim 13 wherein in step vi each solution with the highest fitness is retained in a new solution set for the next generation using selection operations invoking adaptive operations to avoid local optima by removing the other solutions from the solution population S and replacing them by randomly generated solutions.

15. The method of any preceding claim wherein all adaptation operations are executed by the distributed system by monitoring all instances of the algorithm.

16. The method of any preceding claim wherein the markers are regulatory markers.

17. A system for detecting cell types and their associated markers comprising means for executing the following steps:

    a. running a plurality of instances of an algorithm in a distributed system wherein each instance is configured according to the following steps:

        i. receiving a two-dimensional input matrix wherein each vertical vector of the input matrix represents a single cell and each value in the vector represents a feature expression corresponding to that single cell;
        ii. initialising a parent solution set $S_1, S_2, .... , S_n$ from the matrix, where n is a predefined finite number, wherein the solution set comprises a plurality of solutions $S_1, S_2, .... , S_n$ derived from the matrix, wherein each solution $S_i$ has a set of randomly assigned cluster sets $C_1, C_2, ... C_k$ of single cells, where n is the total number of clusters in a given solution;
        iii. computing the fitness of each solution $S_i$ in the solution set using a fitness function, wherein the fitness function is derived from a plurality of objectives;
        iv. applying genetic operators to the solution set to produce an offspring solution set;
        v. computing the fitness of each solution in the offspring set using the fitness function;
        vi. comparing the fitness of each solution in the solution set and the offspring set, wherein each solution with the highest fitness is retained in a new solution set for the next generation;
        vii. repeating steps iii, iv, v and vi for a predetermined number of generations;

    b. selecting the solution set with the highest fitness of the plurality of instances;
    c. extracting the single cell clusters and features from the selected solution set.

100

```
┌─────────────────────┐
│   Single Cell       │
│ Transcriptome or    │ 110
│ Proteome (high      │
│ dimensional raw count│
│ or log₂) Matrix M   │
└─────────────────────┘
          │
          ▼
┌─────────────────────┐
│ Initialize Parent   │ 120
│ Population Set Sₙ    │
└─────────────────────┘
          │
          ▼
┌─────────────────────┐
│ Compute Fitness     │ 130
│ F(Sᵢ) Based on      │
│ Objective 1 and 2   │
└─────────────────────┘
          │
          ▼
┌─────────────────────┐
│ Crossover & Mutation│ 140
│ to Produce new      │
│ Offspring set (O)   │
│ (update adaptation A &│
│ B)                  │
└─────────────────────┘
          │
          ▼
┌─────────────────────┐
│ Compute Fitness for │ 150
│ new offspring set (O)│
│ Based on Objective 1 &│
│ 2                   │
└─────────────────────┘
          │
          ▼
┌─────────────────────┐
│ Selection of new Parent│ 160
│ set (S) from old Parent│
│ (S) and Offspring set │
│ (O)                 │
│ (update adaptation A &│
│ B)                  │
└─────────────────────┘
          │
          ▼
┌─────────────────────┐
│ Termination         │ 170
│ Conditions          │
└─────────────────────┘
          │
          ▼
┌─────────────────────┐
│ Terminate           │ 199
└─────────────────────┘
```

FIG 1B

Single Cell Transcriptome or Proteome (high dimensional raw count or log₂) Matrix *M* — 110

Initialize Parent Population Set $S_n$ — 120

Compute Fitness $F(S_i)$ Based on Objective 1 and 2 — 130

Crossover & Mutation to Produce new *Offspring set (O)* *(update adaptation A & B)* — 140

Compute Fitness for new offspring set (*O*) Based on Objective 1 & 2 — 150

Selection of new Parent set (*S*) from old Parent (*S*) and Offspring set (*O*) *(update adaptation A & B)* — 160

Termination Conditions — 170

Terminate — 199

*gen* ++

No

Yes

FIG. 1A

FIG 1A

100
Instances

Distributed Computing
Algorithm Instances (I)

Best
Solution $S_i$ ⌐180

Extract Single
Cell Clusters,
Markers and Cell
Trajectories ⌐190

FIG. 1B

200

210

220

230

Quality
Control

Fig. 2B

FIG. 2A

**Matrix *M***

FIG. 2B

**Random Solution set $S_n$**

$G_1, G_2, \ldots\ldots G_n$

S

$C_1, \quad C_2, \ldots\ldots\ldots C_n$

$G_1, G_2, \ldots G_n$

S

$C_1, \quad C_2, \ldots\ldots C_n$

$G_1, G_2, \ldots\ldots G_n$

S

$C_1, \quad C_2, \ldots\ldots\ldots C_n$

FIG. 2C

Crossover

FIG. 3

FIG. 4

PBMC

FIG. 5A

## PBMC

FIG. 5B

# BRAIN

## FIG. 5B

## BRAIN

Fitness vs. Number of generations

## FIG. 5C

Protein

FIG. 5E

## Protein

FIG. 5F

FIG. 6A

FIG. 6B

FIG. 6C

PBMC

FIG. 7

PBMC

FIG. 8

PBMC

FIG. 9A

PBMC

P-val=0.167

FIG. 9B

Europäisches
Patentamt

European
Patent Office

Office européen
des brevets

## EUROPEAN SEARCH REPORT

Application Number

EP 22 19 7848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NGUYEN HUNG ET AL: "MGKA: A genetic algorithm-based clustering technique for genomic data", 2019 IEEE CONGRESS ON EVOLUTIONARY COMPUTATION (CEC), IEEE, 10 June 2019 (2019-06-10), pages 103-110, XP033592015, DOI: 10.1109/CEC.2019.8790225 [retrieved on 2019-08-06] * abstract * * page 103, column 1, paragraph 2 - paragraph 3 * * page 107, column 1, paragraph 1 - column 2, paragraph 3 * * page 104, column 1, paragraph 2 * * page 105, column 1, paragraph 2 - column 2, paragraph 2 * * page 106, column 1 * * page 108, column 2, paragraph 3 * | 1-17 | INV. G16B40/30 |
| A | Aliee Hananeh ET AL: "AutoGeneS: Automatic gene selection using multiobjective optimization for RNA-seq deconvolution", Cell Systems, 21 July 2021 (2021-07-21), pages 706-715, XP093026522, Retrieved from the Internet: URL:https://mediatum.ub.tum.de/doc/1639490/document.pdf [retrieved on 2023-02-23] * abstract * * page 707, column 1, paragraph 2 * | 1-17 | TECHNICAL FIELDS SEARCHED (IPC) G16B |

-/--

The present search report has been drawn up for all claims

| Place of search | Date of completion of the search | Examiner |
|---|---|---|
| Munich | 24 February 2023 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 19 7848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MONDAL PRONOY KANTI ET AL: "PseudoGA: cell pseudotime reconstruction based on genetic algorithm", NUCLEIC ACIDS RESEARCH, vol. 49, no. 14, 20 August 2021 (2021-08-20), pages 7909-7924, XP093025702, GB ISSN: 0305-1048, DOI: 10.1093/nar/gkab457 Retrieved from the Internet: URL:https://www.ncbi.nlm.nih.gov/pmc/artic les/PMC8661435/pdf/gkab457.pdf> * abstract * ----- | 1-17 | |

TECHNICAL FIELDS
SEARCHED (IPC)

The present search report has been drawn up for all claims

| Place of search | Date of completion of the search | Examiner |
|---|---|---|
| Munich | 24 February 2023 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ...............................................................................................
& : member of the same patent family, corresponding
    document

EPO FORM 1503 03.82 (P04C01)